# EUROPEAN PATENT SPECIFICATION

(11) **EP 2 101 835 B1**
(45) Date of publication and mention of the grant of the patent: **11.08.2010**
(21) Application number: 08702111.9
(22) Date of filing: 15.01.2008
(51) Int. Cl.: A61L 27/30, A61L 27/54

(54) **METAL IMPLANTS**
METALLIMPLANTATE
IMPLANTS MÉTALLIQUES

(30) Priority: 15.01.2007 GB 0700713; 05.02.2007 GB 0702040
(43) Date of publication of application: 23.09.2009
(73) Proprietor: Accentus Medical plc, 3rd Floor, 11 Strand London WC2N 5HR (GB)
(72) Inventor: PRENTICE, Thomas Campbell, Andover Hampshire SP10 5JL (GB); DAVID, Richard, Lewis, Oxfordshire OX14 1XA (GB); TURNER, Andrew, Derek, Oxon OX14 1XR (GB); PICKFROD, Martin, Edward, Lee, Hants SO31 6WB (GB)
(74) Representative: Mansfield, Peter Turquand
(86) International application number: PCT/GB2008/050028
(87) International publication number: WO 2008/087448

(56) References cited:
- WO-A-00/64505
- WO-A-01/12246
- WO-A-2005/087982
- D. MARTINI ET AL.: "DETACHMENT OF TITANIUM AND FLUOROHYDROXYAPATITE PARTICLES IN UNLOADED ENDOSSEOUS IMPLANTS." BIOMATERIALS, vol. 24, no. 7, 2003, pages 1309-1316, XP002483298
- CHEMICAL ABSTRACTS, vol. 54, no. 2, 2007, Columbus, Ohio, US; abstract no.: 2007:1347253, CHEN, YIKAI ET AL.: "PROSTHETIC IMPLANT ALLOYS COATED WITH ANTIBACTERIAL TITANIUM" XP002483299 & CN 101 073 675 A (SHANGHAI INSTITUTE OF CERAMICS, CHINESE ACADEMY OF SCIENCES, CHINA) 21 November 2007 (2007-11-21)

## Description

This invention relates to metal implants for use in surgical procedures where the implant is to be at least in partly in contact with bone, and in particular to the introduction of a biocidal material into such implants to suppress or control infection, and to a method of making such implants.

Various surgical procedures require the use of implants. A relatively common surgical procedure of this type is hip replacement wherein the head of the femur is partially or fully replaced to remedy imperfections due to wear or disease. In another procedure cancerous bone may be removed, in prosthetic surgery, to be replaced by a metal implant. Such implants may for example be of titanium alloy, which is very strong and relatively light. If part of the implant is to be movable relative to adjacent parts of the body then it is known to provide a smooth and polished surface on that part; and where part of the implant is to be embedded in bone it is known to provide a roughened surface to enhance bone growth onto the implant. A suitably roughened surface may be attained by providing a thermally sprayed coating containing hydroxyapatite to enhance growth of bone on to the implant, and/or by plasma-spraying powdered metal onto the surface.

A potential problem with any such implant is the risk of infection. As described in WO 2005/087982 a titanium metal implant can be treated to form a surface layer that is integral with the metal substrate and which incorporates a biocidal material. The method comprises anodising the implant in phosphoric acid at a voltage above 50 V for a period of at least 30 minutes, so as to generate a surface layer, and then performing ion exchange so as to incorporate ions of a biocidal metal into the surface layer. In instances where an implant is to be located in a region where bone growth is not required (e.g. in contact with or in the vicinity of moving muscles), the surface is preferably polished prior to the anodising treatment. Anodising with the specified electrolyte and specified current density generates a hard surface coating of titania typically of thickness about 0.14 µm, but in which there are pits of diameter up to about 5 µm and depths from about 0.4-3 µm which are filled with titanium oxide (or titanium phosphate). Silver ions can then be incorporated, primarily in the material in these pits, to provide the required biocidal effect. A method of introducing such silver ions while avoiding the need for an anodising step would be advantageous.

WO 01/12246 describes medical devices having a biocompatible coating and containing an antimicrobial metal such as silver. The processes for applying the metal coating can be broadly classified as solution based approaches or vapour phase approaches. D. Martini et al., Biomaterials 24 (2003) pages 1309-1316, describe titanium screws that were subjected to a plasma-spray coating using titanium powders and screws with an additional coating of fluorohydroxyapatite.

According to the present invention there is provided an implant, the implant comprising a metal structure, wherein at least part of the surface of the implant incorporates a coating of a biocompatible metal deposited by plasma-spraying a powder of the biocompatible metal, and the coating incorporates biocidal metal cations in the coating.

The present invention also provides a method of making an implant, the implant comprising a metal structure, the method comprising the steps of plasma spraying a powder of a biocompatible metal on to at least part of the surface of the metal structure so as to form a coating of the biocompatible metal, and then contacting the coating with a solution containing a biocidal metal, such that cations of the biocidal metal are incorporated into the coating.

It is believed that the biocidal cations are incorporated by an ion-exchange process.

That a metal surface deposited by plasma spraying should have ion exchange properties is surprising. It is hypothesised that the ion exchange properties are due to oxide originally on the surface of the individual particles of the powder prior to its deposition. Sufficient biocidal ions can be adsorbed to provide the requisite biocidal effect for a prolonged period, for example for at least six weeks and more preferably for at least six months after implantation, with a low release rate to avoid toxic effects on body cells.

Depending on the material of which the metal structure is made, the plasma-coated structure may then be anodised prior to contact with the solution containing the biocidal metal. It has been found that the anodised coated structure generally incorporates decreased amounts of biocidal metal.

In a modification of the process the deposited coating is first contacted with phosphoric acid (which may convert at least some surface oxide to phosphate); the surface is then rinsed before being contacted with the solution containing the biocidal metal. The rinsing step removes displaceable phosphate ions. The phosphate acid-treated surface generally incorporates decreased amounts of biocidal metal.

In principle, a range of different materials may be used for the biocidal material. Gold, platinum and palladium would be potentially suitable, although expensive; silver is preferable as it is not particularly soluble in body fluids owing to the presence of chloride ions and the low solubility of silver chloride.

The loading of silver ions in the coating of the finished implant may be in the range of from 1 to 100 µg/cm², e.g., from 2 to 20 µg/cm².

Other elements such as copper, tin, antimony, lead, bismuth and zinc might be used as ions combined into the surface layer. The rate of release would be controlled, in this case, primarily by the strength of the bonding forces of the metal ions in the layer.

The metal structures of such prosthetic implants are typically of a form of stainless steel, a titanium alloy, a cobalt/chromium alloy, or alloys or metals based on niobium, tantalum or zirconium. Suitable standard alloys for prosthetic implants are titanium 90% with 6% aluminium and 4% vanadium (British standard 7252), or chromium 26.5-30%, molybdenum 4.5-7%, and the remainder cobalt (British standard 7252 part 4). The coating may be of a titanium alloy, or it may be a pure metal such as titanium, niobium or tantalum, or alloys of these metals.

The biocompatible metal powder may be oxidatively pre-treated prior to the plasma spraying step in order to increase the oxide content of the plasma-sprayed coating. An increase in the oxide content of the plasma-sprayed coating has been found to increase the amount of biocidal metal which can be incorporated into the surface of the coating. The oxidative pre-treatment may be thermal (e.g. heating in air or low O₂/N₂ mixtures under controlled conditions) or by chemical methods such as exposure to an oxidant in solution.

The invention will now be further and more particularly described, by way of example only.

An implant for use as a tibia prosthesis comprises a structure made of cobalt/chromium alloy. The implant structure is of dimensions that are specific for use with a particular patient. At least part of the implant will, when implanted, be in contact with bone, and it is therefore desirable that bone should bond to the surface of that part. This bone bonding process may be helped by providing a rough surface, for example by plasma spraying of titanium powder.

This plasma spraying is a conventional process. It would typically use titanium powder of particle size in the range 30 to 200 µm. To prevent significant oxidation of the powder (which is highly reactive when hot) the plasma spraying would typically use an argon/2% hydrogen plasma, and the powder is sprayed at high velocity through this plasma to impact with the surface. The spraying process may take place in an evacuated chamber, but would more typically be carried out in a chamber containing air. The plasma gases prevent significant contact between the air in the chamber and the hot metal powder, although there may be small amounts of adventitious oxygen which would react with the deposited metal.

If the metal structure of the implant is titanium, niobium, tantalum, zirconium or a suitable alloy of these metals, the plasma-sprayed implant may be anodised in the known manner after the plasma spraying step. It has been found that anodised plasma-sprayed implants incorporate increased amounts of biocidal metal.

Alternatively, the plasma-sprayed implant may be contacted with dilute phosphoric acid to convert at least some of the surface titanium oxide to phosphate, and then preferably rinsed to remove excess phosphate ions.

The coated implant is then contacted with a solution containing silver cations so that these are adsorbed into the oxide/phosphate surface. Preferably, suitable steps may be taken to promote wetting of the coated implant by the silver salt solution. For example, a vacuum may be applied to the solution during contact with the coated implant to displace air bubbles trapped in surface asperities, and/or a non-ionic surfactant may be added to or included in the silver salt solution. The silver salt solution may be stirred to promote uniform incorporation of silver cations in the surface of the plasma-coated implant. In one example, after soaking in 20 wt% phosphoric acid for 2 hours, and then immersion in 0.01 M silver nitrate for 2 hours, the silver loading was 2.4 µg/cm².

In a slightly simpler process the pretreatment with phosphoric acid is not performed, the as-deposited coating being contacted with the solution of the silver salt. This simpler process can also enable sufficient silver to be incorporated. Immersion in 0.01M silver nitrate for 2 hours gave a subsequent silver loading of 3.7 µg/cm², while a similar immersion in 0.05M silver nitrate gave a loading of 13.3 µg/cm².

After the implant is implanted into a patient, silver ions are gradually leached out into the surrounding body fluids, so that any bacteria in the immediate vicinity of the implant are killed. Infection arising from the implant is therefore suppressed.

It will be appreciated that the surface that is coated by plasma spraying with titanium may be pretreated in various ways, for example it would typically be thoroughly cleaned, and may also be shot blasted to provide a rough surface to provide a strong bond.

In a further modification the treated titanium coating (containing biocidal metal, e.g. silver ions) might then be coated with hydroxyapatite, which may be further treated with a solution containing biocidal metal (e.g. treated with dilute silver salt solution) to load or incorporate additional biocidal metal ions, such as silver ions, into the hydroxyapatite.

As described above the titanium coating may be deposited on to a structure of cobalt/chromium alloy, but the titanium coating may equally be deposited on to structures of other metals including titanium alloy. And the coating itself may also be of other metals, for example of cobalt/chromium alloy.

## Claims

1. An implant, the implant comprising a metal structure, wherein at least part of the surface of the implant incorporates a coating of a biocompatible metal deposited by plasma-spraying a powder of the biocompatible metal, and the coating incorporates biocidal metal cations adsorbed into the surface of the coating.

2. The implant of claim 1 wherein the biocidal metal cations comprise silver ions.

3. An implant as claimed in claim 2 wherein the loading of silver ions in the coating is from 1 to 100 µg/cm².

4. An implant as claimed in any of claims 1 to 3 wherein the plasma coated structure is either anodized or treated with phosphoric acid.

5. The implant of any of claims 1 to 4 wherein the plasma-coated structure containing biocidal metal is coated with hydroxyapatite, and optionally, additional biocidal metal cations are loaded into the hydroxyapatite.

6. A method of making an implant, the implant comprising a metal structure, the method comprising the steps of plasma spraying a powder of a biocompatible metal on to at least part of the surface of the metal structure so as to form a coating of the biocompatible metal, and then contacting the coating with a solution containing a biocidal metal, such that cations of the biocidal metal are adsorbed into the surface of the coating.

7. A method as claimed in claim 6 wherein the coating is first contacted with phosphoric acid before being contacted with the solution containing the biocidal metal.

8. A method as claimed in claim 6 wherein the coated structure is anodized prior to contacting with the solution of biocidal metal.

9. A method as claimed in any of claims 6 to 8 wherein the plasma-coated structure containing biocidal metal is coated with hydroxyapatite, and optionally, the hydroxyapatite-coated structure is treated with a solution containing biocidal metal cations to incorporate at least some of the latter biocidal metal cations into the hydroxyapatite coating.

10. A method as claimed in any of claims 6 to 9 wherein the biocidal metal is silver.

## Patentansprüche

1. Metallstruktur aufweisendes Implantat
**dadurch gekennzeichnet,**
**dass** zumindest ein Teil der Oberfläche des Implantats eine Beschichtung aus biokompatiblem Metal aufweist, welche durch Plasmaspritzen eines Pulvers aus biokompatiblem Metal abgeschieden wird, wobei die Beschichtung biozide Metallkationen aufweist, die in die Oberfläche der Beschichtung adsorbiert sind.

2. Implantat nach Anspruch 1
**dadurch gekennzeichnet,**
**dass** die bioziden Metallkationen Silberionen aufweisen.

3. Implantat nach Anspruch 2
**dadurch gekennzeichnet,**
**dass** die Beladung der Beschichtung mit Silberionen zwischen 1 und 100 µg/cm² beträgt.

4. Implantat nach einem der Ansprüche 1 bis 3,
**dadurch gekennzeichnet,**
**dass** die plasmabeschichtete Struktur entweder eloxiert ist oder mit Phosphorsäure behandelt ist.

5. Implantat nach einem der Ansprüche 1 bis 4,
**dadurch gekennzeichnet,**
**dass** die plasmabeschichtete Struktur, welche das biozide Metall umfasst mit Hydroxylapatit beschichtet ist, wobei das Hydroxylapatit optional mit zusätzlichen biozidalen Metallkationen beladen ist.

6. Verfahren zur Herstellung eines eine Metallstruktur aufweisenden Implantats, wobei des Verfahren die Schritte Plasmaspritzen eines Pulvers aus biokompatiblem Metall auf zumindest einen Teil der Oberfläche der Metallstruktur, so dass eine Beschichtung aus biokompatiblem Metall gebildet wird, und Kontaktieren der Beschichtung mit einer biozidales Metall enthaltenden Lösung umfasst, so dass Kationen des biozidalen Metalls in die Oberfläche der Beschichtubg adsorbiert werden.

7. Verfahren nach Anspruch 6
**dadurch gekennzeichnet,**
**dass** die Beschichtung vor dem Kontaktieren mit der biozidales Metall enthaltenden Lösung zuerst mit Phosphorsäure kontaktiert wird.

8. Verfahren nach Anspruch 6
**dadurch gekennzeichnet,**
**dass** die beschichtete Struktur vor dem Kontaktieren mit der biozidales Metall enthaltenden Lösung eloxiert wird.

9. Verfahren nach einem der Ansprüche 6 bis 8
**dadurch gekennzeichnet,**
**dass** die plasmabeschichtete, biozidales Metall aufweisende Struktur mit Hydroxylapatit beschichtet wird, wobei die hydroxylapatitbeschichtete Struktur optional mit einer biozidale Metallkationen aufweisenden Lösung zur Aufnahme von zumindest einigen der letzteren biozidalen Metallkationen in die Hydroxylapatitbeschichtung behandelt wird.

10. Verfahren nach einem der Ansprüche 6 bis 9
**dadurch gekennzeichnet,**
**dass** es sich bei dem biozidalen Metall um Silber handelt.

## Revendications

1. Implant, l'implant comprenant une structure métallique, dans lequel au moins une partie de la surface de l'implant incorpore un revêtement d'un métal biocompatible déposé par projection au plasma d'une poudre du métal biocompatible, et le revêtement incorpore des cations de métal biocide adsorbés dans la surface du revêtement.

2. Implant selon la revendication 1, dans lequel les cations de métal biocide comprennent des ions d'argent.

3. Implant selon la revendication 2, dans lequel la charge d'ions d'argent dans le revêtement va de 1 à 100 µg/cm².

4. Implant selon l'une quelconque des revendications 1 à 3, dans lequel la structure revêtue au plasma est soit anodisée soit traitée avec de l'acide phosphorique.

5. Implant selon l'une quelconque des revendications 1 à 4, dans lequel la structure revêtue au plasma contenant un métal biocide est revêtue d'hydroxyapatite et, facultativement, des cations de métal biocide supplémentaires sont chargés dans l'hydroxyapatite.

6. Procédé de fabrication d'un implant, l'implant comprenant une structure métallique, le procédé comprenant les étapes de projection au plasma d'une poudre d'un métal biocompatible sur au moins une partie de la surface de la structure métallique de façon à former un revêtement du métal biocompatible et, ensuite, de mise en contact du revêtement avec une solution contenant un métal biocide, de sorte que les cations du métal biocide sont adsorbés dans la surface du revêtement.

7. Procédé selon la revendication 6, dans lequel le revêtement est d'abord mis en contact avec de l'acide phosphorique avant d'être mis en contact avec la solution contenant le métal biocide.

8. Procédé selon la revendication 6, dans lequel la structure revêtue est anodisée avant d'être mise en contact avec la solution de métal biocide.

9. Procédé selon l'une quelconque des revendications 6 à 8, dans lequel la structure revêtue au plasma contenant un métal biocide est revêtue d'hydroxyapatite et, facultativement, la structure revêtue d'hydroxyapatite est traitée avec une solution contenant des cations de métal biocide de façon à incorporer au moins certains de ces derniers cations de métal biocide dans le revêtement d'hydroxyapatite.

10. Procédé selon l'une quelconque des revendications 6 à 9, dans lequel le métal biocide est de l'argent.
